# EUROPEAN PATENT SPECIFICATION

(11) **EP 2 565 187 B1**
(45) Date of publication and mention of the grant of the patent: **17.09.2014**
(21) Application number: 12460016.4
(22) Date of filing: 13.04.2012
(51) Int. Cl.: C07D 251/54, D06M 13/358

(54) **New reactive triazine derivatives as ultraviolet absorbers increasing barrier properties of cellulose fibers and their preparation**
Neue reaktive Triazinderivate als Ultraviolet-Absorptionsmittel zur Erhöhung der Sperreigenschaften von Cellulosefasern sowie deren Herstellung
Nouveaux dérivés de triazine comme absorbeurs d'UV réactifs améliorant les propriétés de barrière des fibres de cellulose et leur préparation

(30) Priority: 19.04.2011 PL 39461611
(43) Date of publication of application: 06.03.2013
(73) Proprietor: Instytut Wlokiennictwa, 92-103 Lodz (PL)
(72) Inventor: Sójka-Ledakowicz, Jadwiga, 91-164 Lodz (PL); Lewartowska, Joanna, 90-255 Lódz (PL)
(74) Representative: Wroblewski, Michal

(56) References cited:
- DE-A1- 19 720 683

## Description

The subject matter of the invention relates to novel reactive UV absorbers which increase the barrier properties of the cellulose fibers and the method in which they are produced.

In the last decades, increased attention has been paid to the harmful effects ultraviolet radiation has on human skin. Civil unrests publicized by the media have been further intensified by the fears of scientists dealing with the disappearance of the ozone layer in the atmosphere. As a result of this phenomenon, which is also aided by active human industrial activity, more and more high-energy ultraviolet radiation fractions reach the surface of the Earth.

The results of numerous medical studies confirm that the component of UV-B ultraviolet radiation is the main factor responsible for the direct changes in the structure of DNA which are caused by photochemical processes and may result in skin cancer. Radiation in this area has been shown to produce erythema, with the largest changes observed in the wavelength range of 305-310 nm. This activity also causes skin burns, and its chronic effects are premature senile changes and reduced immunity.

The UV-A range of ultraviolet radiation, which, among other things, initiates the formation of a tan, is less harmful, but excessive exposure to it can also be dangerous, especially for people with sensitive skin. It causes loss of skin elasticity, promotes the formation of wrinkles and increases susceptibility to various allergic reactions [S.Lautenschlager, HCWulf, MRPitelkow, The Lancet 370, 528-535 (2007)].

In light of the considerations listed above, skin protection becomes increasingly important. This protection is provided by textiles, which in practice have indeed been the oldest agents used for this purpose. Also, today the primary recommendation of the WHO in the field of protection against ultraviolet radiation is to wear loose clothing made of tightly woven fabric, covering the largest possible surface of the body.

The belief that all textile products offer sufficient protection of the skin from sunlight is wrong, because this property is the result of many factors such as the chemical structure of a particular type of fiber, the structural characteristics of the sample (porosity, mass per unit area, surface weight, thickness, flexibility), the color and intensity of the color, and a particular product finish.

Among the textiles used in practice, cellulose fiber products protect the human skin the least. Clothing made of thin cotton or viscose yarn of white or pastel colors, which, for aesthetic and practical reasons, are the most popular in the summertime, are characterized by very weak protective properties with low values of the UPF (Ultraviolet Protection Factor) coefficient. This coefficient, used in practice as the measure of barrier properties of textiles against ultraviolet radiation, indicates the difference between the time a person can remain in the sun with or without protective clothing. According to the current European standard [EN 13758-1. Solar UV-protection properties, part 1 - Method of test for apparel fabrics (2001)] the value of UPF > 40 is considered sufficient.

One of the ways to improve the barrier properties of textile products is the use of special UV absorbers that contain reactive groups for covalent fiber bonding. Chemical bonding of the UV absorber with the fiber, though requiring special conditions for the application, allows for long-term retention of the barrier properties of the product in spite of repeated washing operations [W.Czajkowski, J.Paluszkiewicz, Przemysl Chemistry, 87 (10), 1029-1034 (2008)].

Among the compounds which raise the barrier properties of textile materials containing cellulose fibers, there are derivatives of 1,3,5-triazine. Examples of such substances are described in a number of patents presenting new compounds (U.S. 5,786,475, DE 19720683, DE 19509670), methods of their production (U.S. 5,786,475, DE 19509670, CA 2145268, DE 19509670) and the use (U.S. 5,786,475, DE 19509670, DE 19720,683, U.S. 5938793, DE 19509670, WO9625549, U.S. 5,998,306).

In the course of the research it has been found that the introduction of additional triazine residues to the UV absorber molecule causes a significant increase in its molar absorption coefficient. Such products allow for obtaining high values of UPF at lower concentrations of absorber in the application bath, thereby reducing the amount of chemical substances entering the textile product.

The essence of the solution according to the invention relates to novel reactive ultraviolet absorbers in the form of derivatives of symmetric 1,3,5-triazine with the following formula 1: 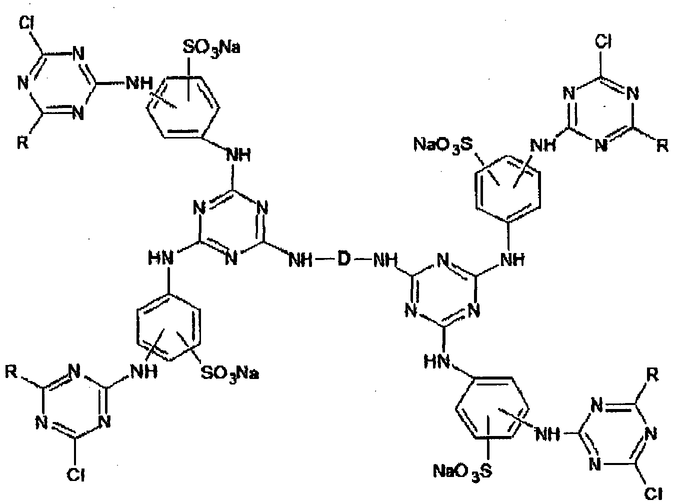 in which D represents a single bond or an aliphatic or aromatic diamine residue, R represents chlorine atom, methoxy residue, aminophenyl-sulphonic acid residue, aminophenyl disulphonic residue, ethanolamine or diethanolamine residue, morpholine residue, or residue represented by formula II:

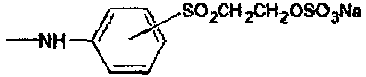

According to the invention, in the method of obtaining a new group of absorbers, dispersed 2,4,6-trichloro-1,3,5-triazine is condensed with aryl diaminosulphonic acid, preferably 2,5- diaminobenzene sulphonic or 1,4-diaminophenyl-2,5 - disulphonic acid in the ratio of one mole of acid per one mole of triazine. Then, the product of the reaction is condensed with an additional mole of aryl diaminosulphonic acid. The resulting intermediate is condensed with aromatic diamine, preferably p-phenylenediamine, o-phenylenediamine or hydrazine, used in quantities of half mole of diamine per mole of the reaction product. Finally condensation product is condensed with four moles of chlorinated derivatives of symmetric 1,3,5-triazine.

The invention has been illustrated by examples shown which do not in any way limit the scope of protection.

### Example 1.

15.6 g of 2,4,6-trichloro-1 ,3,5-triazine was dispersed in a mixture of 60 cm³ of water, 140 g of ice and a few drops of Rokacet 07 dispersant. Then, with constant stirring, a solution of 15 g of 2,5-diaminobenzenesulphonic acid in 50 cm³ of water containing 20 cm³ of 10% sodium hydroxide solution to pH = 7.0 (± 0.2) was added. Condensation was carried out at the temperature of 0-5° C while maintaining the value of pH = 6.0-6.5 by addition of 32 cm³ of 10% sodium hydroxide solution. After 1.5 hours TLC chromatography analysis showed the disappearance of 2,5- diaminobenzene sulfonic acid in the reaction mixture. After the completion of the first condensation, 13.2 g of 2,5-diaminobenzenesulphonic acid dissolved in 25 cm³ of water containing 20 cm³ of 10% sodium hydroxide solution to pH = 7 was added, the temperature of the reaction mixture was raised to 30-35 ° C and stirred for additional 4 hours maintaining the pH = 6.5 ± 0.2 by adding 24 cm³ of 10% sodium hydroxide solution. The completion of the reaction was determined by TLC chromatography using Kieselgel 60 F254 plates and developing system n-butanol, n-propanol, ethyl acetate, water, in the ratio of 2:4:1:3.

After obtaining the complete overreaction of 2,5- diaminobenzenesulphonic acid, the reaction mixture was heated to the temperature of 55° C and then a solution of 4.1 g p-phenylenediamine dissolved in 50 cm³ of warm water with I g of Na₂SO₃ was added. Within 30 minutes the reaction mixture was heated to the temperature of 75° C, allowing the pH to drop to about 3.5-4, then the pH was raised to 5.5 by adding 10 cm³ of 10% sodium hydroxide solution and the mixture was heated for additional 30 minutes at 80 - 85°C, after which the pH was raised to 7.5 by the addition of 8 cm³ of 10% sodium hydroxide solution and under these conditions the mixture was stirred for additional 2.5 hours. The reaction product was isolated by the gradual addition of 250 cm³ of 2% hydrochloric acid (to pH about 1.5), and then filtered. After drying, 44.8 g of product containing 73.9% of the intermediate "tetra-amine" (analysis by titration with standard solution of sodium nitrite) was obtained, which equaled 81.9% of theoretical efficiency.

13.66 g of the intermediate product (the amount corresponding to 10.1 g of pure amine) was dissolved in 100 cm³ of water containing 20 cm³ of 10% sodium hydroxide solution to pH = 6.5 (± 0.2) and, added to a suspension prepared as previously of 7.4 g of 2,4,6-trichloro-1 ,3,5-triazinc in 30 cm³ of water and 70 g of ice with a few drops of Rokacet 07 dispersant. Condensation was carried out at the temperature of 0-5°C while maintaining pH 6.0-6.5 by addition of 13 cm³ of 10% sodium hydroxide solution.

After two hours, the Ehrlich's reagent test showed the disappearance of the free amine in the reaction. The product was isolated by salting out with sodium chloride added in the amount of 100 g, which is 20% in proportion to the volume of the reaction mixture, and filtered. The paste was mixed with 10 cm³ of phosphate buffer of pH 7 and dried at the temperature not exceeding 35°C.

41.8 g of formula III product was obtained in the form of gray powder containing

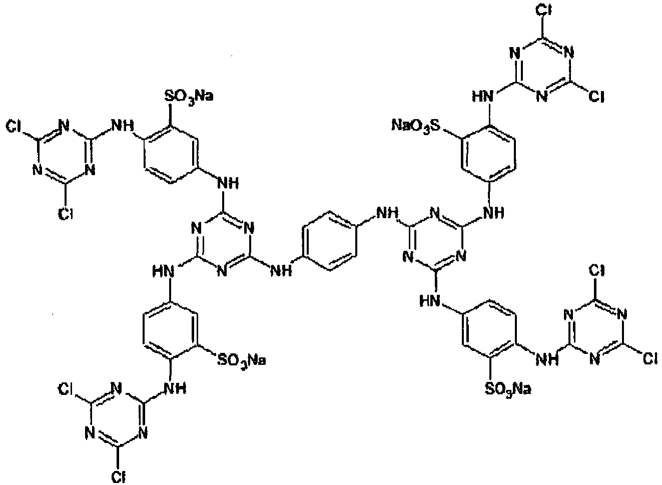

52.86% of sodium chloride, and 14.08% of reactive chlorine in the form of dichlorotriazine residue, estimated by the conductometric analysis of the sample before and after alkaline hydrolysis with standard AgNO₃ solution. Mass spectrum of the product was made, which confirmed its structure (peak [M-Na 2 H] + m / z = 1665 peak [M+3Na]³⁺ m / z = 585). In aqueous solutions the product, free from inorganic salts, showed λₘₐₓ = 300-305 nm and εmol≈90000-100000.

### Example 2

5.6 g of 2,4,6-trichloro-1,3,5-triazine was dispersed in a mixture of 60 cm³ of water, 140 g of ice and a few drops of Rokacet 07 dispersant Then, with constant stirring, a solution of 15 g of 2,5-diaminobenzenesulphonic acid in 50 cm³ of water containing 20 cm³ of 10% sodium hydroxide solution to pH = 7.0 (± 0.2) was added. Condensation was carried out at the temperature of 0-5 ° C while maintaining the pH = 6.0-6.5 by addition of 32 cm³ of 10% sodium hydroxide solution. After 1.5 hours TLC chromatography analysis showed the disappearance of 2,5- diaminobenzenesulphonic acid in the reaction mixture. After the conclusion of the first condensation, 13.2 g of 2,5- diaminobenzene sulfonic acid dissolved in 25 cm³ of water containing 20 cm³ of 10% sodium hydroxide solution to pH = 7 was added, the temperature of the reaction mixture was raised to 30-35° C and stirred for additional 4 hours maintaining the pH = 6.5 ± 0.2 by adding 24 cm³ of 10% sodium hydroxide solution. The completion of the reaction was determined by TLC chromatography using Kieselgel 60 F254 plates and developing system: n-butanol, n-propanol, ethyl acetate, water, in the ratio of 2:4:1:3. After obtaining the complete overreaction of 2,5- diaminobenzenesulphonic acid, the reaction mixture was heated to the temperature of 55° C and then a solution of 4.1 g o-phenylenediamine dissolved in 50 cm³ of water with 6 cm³ of 30% hydrochloric acid was added. Within 30 minutes the reaction mixture was heated to the temperature of 75° C, allowing the pH to drop to about 3.5-4, then the pH was raised to 5.5 by adding 10 cm³ of 10% sodium hydroxide solution and the mixture was heated for additional 30 minutes to 80 - 85° C, after which the pH was raised to 7.5 by the addition of 8 cm³ of 10% sodium hydroxide solution and under these conditions the mixture was stirred for additional 2.5 hours. The reaction product was isolated by the gradual addition of 250 cm³ of 2% hydrochloric acid (to pH about 1.5), and then filtered. After drying, 40.2 g of product containing 79.2% of the intermediate "tetra-amine" (analysis by titration with standard solution of sodium nitrite) was obtained, which equaled 78.8% of theoretical efficiency.

12.7 g of the above product (the amount corresponding to 10.1 g of pure amine) was dissolved in 100 cm³ of water containing 16 cm³ of 10% sodium hydroxide solution to pH = 6.5 (± 0.2) and, added to a suspension prepared as previously of 7.4 g of 2,4,6-trichloro-1.,3,5-triazine in 30 cm³ of water and 70 g of ice with a few drops of Rokacet 07 dispersant. Condensation was carried out at the temperature of 0-5°C while maintaining pH 6.0-6.5 by addition of 13 cm³ of 10% sodium hydroxide solution. After two hours, the Ehrlich's reagent test showed the disappearance of the free amine in the reaction. The product was isolated by salting out with sodium chloride added in the amount of 100 g, which is 20% in proportion to the volume of the reaction mixture, and filtered. The paste was mixed with 10 cm³ of phosphate buffer of pH 7 and dried at the temperature not exceeding 35° C.

27.4 g of formula IV product was obtained in the form of gray-violet powder containing

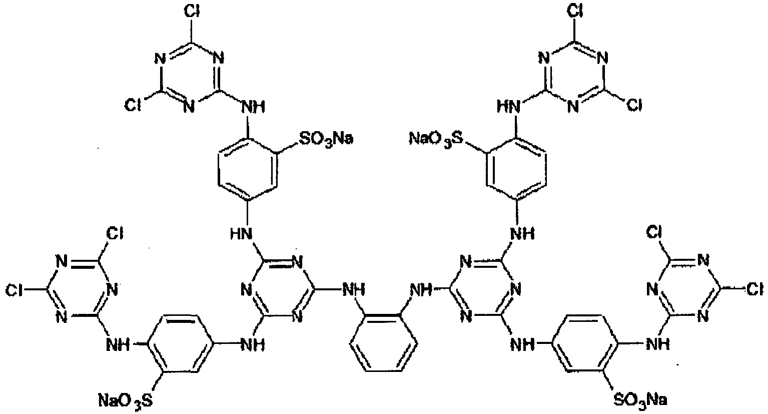

40.73% of sodium chloride, and 7.88% of reactive chlorine in the form of dichlorotriazine residue (the conductometric analysis of the sample before and after alkaline hydrolysis with designated solution of silver nitrate). Mass spectrum of the product was made, which confirmed its structure (peak [M+Na]⁺ m/z=1709. The presence of the hydrolyzed product in the form of peak [M-4Cl+4OH+3H]³⁺ m/z=539) was also observed. In addition, mass spectrum of negative ions was made showing the presence of peak [M-Na-2H]³⁻ m/z=553. In aqueous solutions the product, free from inorganic salts, showed λmax = 293-298 nm and εmol≈ 80000.

### Example 3

15.6 g of 2,4,6-trichloro-1,3,5-triazine was dispersed in a mixture of 60 cm³ of water, 140 g of ice and a few drops of Rokacet 07 dispersant. Then, with constant stirring, a solution of 15 g (99.83%) of 2,5-diaminobenzenesulphonic acid was added in 50 cm³ of water containing 20 cm³ of 10% sodium hydroxide solution to pH = 7.0 (± 0.2) was added. Condensation was carried out at the temperature of 0-5° C while maintaining the pH = 6.0-6.5 by addition of 32 cm³ of 10% sodium hydroxide solution. After 1.5 hours TLC chromatographic analysis showed the disappearance of 2,5- diaminobenzene sulfonic acid in the reaction mixture. After the completion of the first condensation, 13,2 g of 2,5-diaminobenzenesulphonic acid dissolved in 25 cm³ of water containing 20 cm³ of 10% sodium hydroxide solution to pH = 7 was added, the temperature of the reaction mixture was raised to 30-35° C and stirred for additional 4 hours maintaining the pH = 6.5 ± 0.2 by adding 24 cm³ of 10% sodium hydroxide solution. The end-point of the reaction was determined by TLC chromatography using Kieselgel 60 F254 plates and developing system: n-butanol, n-propanol, ethyl acetate, water, in the ratio of 2:4:1:3. After obtaining the complete overreaction of 2,5- diaminobenzenesulphonic acid, the reaction mixture was heated to the temperature of 55° C and then a solution of 4 cm³ 40% hydrazine hydrate was added. The reaction mixture was heated to the temperature of 75° C, stirred at this temperature while maintaining the pH 8.5-9 by adding 6 cm³ of 10% NaOH solution for 2 hours. The reaction product was isolated by gradual addition of 250 cm³ of 2% hydrochloric acid 2% (to pH about 1.5) and then filtered. After drying, 35.2 g of product containing 79.4% of the intermediate "tetra-amine" (analysis by titration with standard solution of sodium nitrite) was obtained, which equaled 74.7% of theoretical efficiency.

11.77 g of the above described product (the amount corresponding to 9.34 g of pure amine) was dissolved in 100 cm³ of water containing 15 cm³ of 10% sodium hydroxide solution to pH = 6.5 (± 0.2) and added to a suspension prepared as previously of 7.4 g of 2,4,6-trichloro-1,3,5-triazine in 30 cm³ of water and 70 g of ice with a few drops of Rokacet 07 dispersant. Condensation was carried out at the temperature of 0-5° C while maintaining pH 6.0-6.5 by addition of 12 cm³ of 10% sodium hydroxide solution. After two hours, the Ehrlich's reagent test showed the disappearance of the free amine in the reaction. The product was isolated by salting out with sodium chloride added in the amount of 100 g, which is 20% in proportion to the volume of the reaction mixture, and filtered. The paste was mixed with 10 cm³ of phosphate buffer of pH 7 and dried at the temperature not exceeding 35° C. 26.4 g of formula V product was obtained

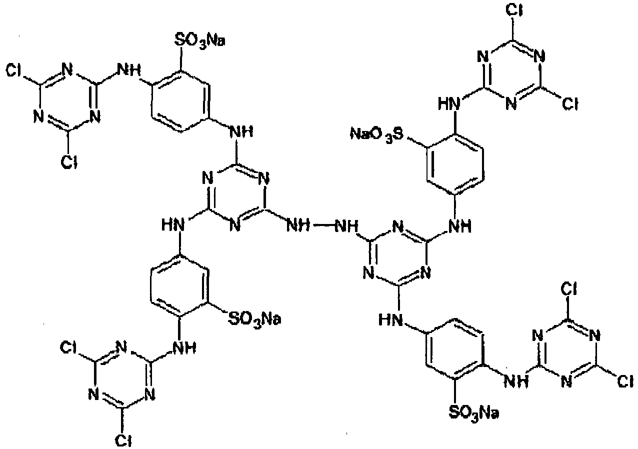

in the form of gray-brown powder containing 36.88% of sodium chloride, and 11.09% of reactive chlorine in the form of dichlorotriazine residue (the conductometric analysis of the sample before and after alkaline hydrolysis with standard solution of AgNO₃). Mass spectrum of the product was made, which confirmed its structure (peak [M+2Na+H]³⁺ m/z=552; peak [M+3Na]³⁺ m/z=560). In addition, mass spectrum of negative ions was made showing the presence of peak [M-Na-2H]³⁻ m/z=528. In aqueous solutions the product, free from inorganic salts, showed λmax = 299 nm and εmol ≈ 80000.

### Example 4

13.66 g of intermediate amine (the amount corresponding to 10.1 g of pure product) obtained as in Example 1, was dissolved in 100 cm³ of water containing 20 cm³ of 10% sodium hydroxide solution to pH = 6.5 (± 0.2) and added to a suspension prepared as previously of 7.4 g of 2,4,6-trichloro-1,3,5-triazine in 30 cm³ of water and 70 g of ice with a few drops of Rokacet 07 dispersant. Condensation was carried out at the temperature of 0-5°C while maintaining pH 6.0-6.5 by addition of 13 cm³ of 10% sodium hydroxide solution. After two hours, the Ehrlich's reagent test showed the disappearance of the free amine in the reaction. A solution of 11.2 g of sodium salt of (4-β-sulphatoethyl) sulphonyl aniline in the mixture of 50 cm³ of hot water and 20 cm³ of 10% sodium carbonate solution was added. The reaction mixture was heated to 40°C and stirred at this temperature while maintaining the pH = 7 by addition of 10 cm³ of 10% sodium hydroxide solution. After reaction end-point the product was isolated by salting out with sodium chloride added in the amount of 200 g, which is 20% in proportion to the volume of the reaction mixture, and filtered. The paste was mixed with 10 cm³ of phosphate buffer of pH 7 and dried at the temperature not exceeding 35° C.

54.6 g of formula VI product was obtained: 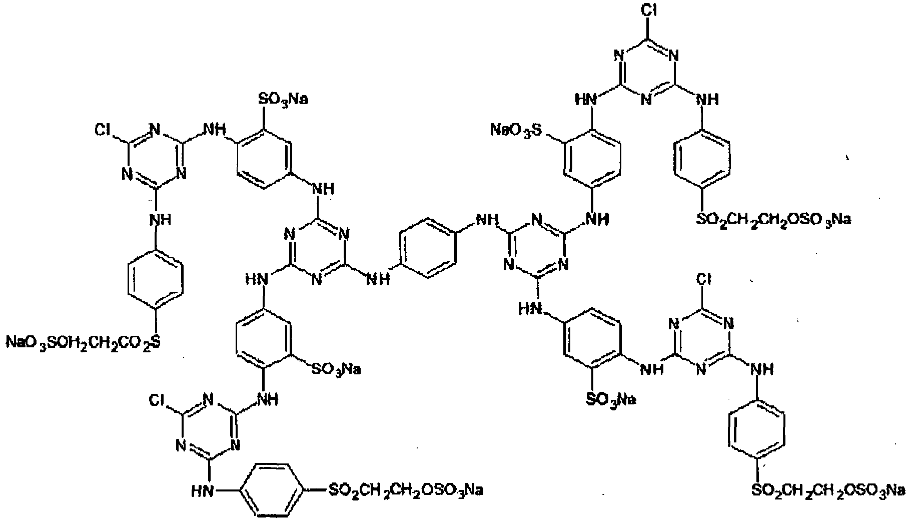 in the form of gray-brown powder containing 55.78% of sodium chloride, and 2.72% of ractive chlorine in the form of monochlorotriazine residue (the conductometric analysis of the sample before and after alkaline hydrolysis with standard solution of silver nitrate).

Mass spectrum of the product was made, which confirmed its structure (peak [M+Na]⁺ m/z=2777; peak [M-4Na+8H]⁴⁺ m/z=668;). In addition, the presence of the hydrolyzed product of the peak [M-4SO₃Na+8H]⁴⁺ m/z-588) was also observed. In aqueous solutions the product, free from inorganic salts, showed λmax = 291nm and εmol ≈ 140 000.

### Example 5

11.77 g of the intermediate product (the amount corresponding to 9.34 g of pure amines) obtained in as in Example 3, was dissolved in 100 cm³ of water containing 15 cm³ of 10% sodium hydroxide solution to pH = 6.5 (± 0.2) and added to a suspension prepared as previously of 7.4 g of 2,4,6-trichloro-1,3,5-triazine in 30 cm³ of water and 70 g of ice with a few drops of Rokacet 07 dispersant. Condensation was carried out at the temperature of 0-5° C while maintaining pH 6.0-6.5 by addition of 12 cm³ of 10% sodium hydroxide solution. After two hours, the Ehrlich's reagent test showed the disappearance of the free amine in the reaction mixture. A solution of 11.2 g of sodium salt (4-β-sulphate ethyl) sulphonyl aniline was added in the mixture of 50 cm³ of hot water and of 20 cm³ of 10% sodium carbonate solution was added. The reaction mixture was heated to 40° C and stirred at this temperature while maintaining the pH = 7 by addition of 8 cm³ of 10% sodium hydroxide solution. After reaction end-point the product was isolated by salting out with sodium chloride added in the amount of 140 g, which is 20% in proportion to the volume of the reaction mixture, and filtered. The wet paste was mixed with 20 cm³ of phosphate buffer of pH 7 and dried at the temperature not exceeding 35° C. 45.8 g of formula VII product was obtained in the form of gray-brown powder containing 42.7 g of sodium chloride, and 6.8% of reactive chlorine in the form of monochlorotriazine residue (the conductometric analysis of the sample before and after alkaline hydrolysis with standard solution of silver nitrate). Mass spectrum of the product was made, which confirmed its structure (peak [M-2Na 6 H] 4 + m / z = 660, peak [M + Na 3 H] 4 + m / z = 676). In addition, the presence of the hydrolyzed product of the peak [M-2SO₃Na+4H]²⁺ m/z=1238) was also observed. In aqueous solutions the product, free from inorganic salts, showed λmax = 289 nm and εmol ≈ 130 000.

### Example 6 - Application of absorbers - formula III, IV or V.

Samples of cotton fabric with the mass of 2 g and surface density of 175 g/m², were placed in a bath containing 30 cm³ of water and 0.02 g of the absorber (1% on the weight of the mass of fibers) or in a bath containing 30 cm³ of water and 0.01 g (0,5% on the weight of the mass of fibers). The application process was carried out at the temperature of 30° C. After 10 minutes, 1.5 g of crystalline sodium (VI) sulphate was added to application bath and application process continued. After 20 minutes, 0.15 g of sodium carbonate was added, and after additional 20 minutes another 0.15 g of sodium carbonate was added. Application was continued for 30 more minutes at 30-40° C, after which the fabric samples were rinsed, washed and dried. Using the spectrophotometer JASCO V-550 equipped with an integrating sphere, UPF coefficient values were determined according to the method described in European Standard BS EN 13758-1:2007. The values of the UPF coefficients of the samples after application bath in solutions containing compounds of formula III, IV or V are given in Table 1.

### Example 7 - Application of absorbers of the formula VI or VII - method 1.

A sample of cotton fabric with the mass of 2 g and surface density of 175 g/m², was placed in a bath containing 30 cm3 of water and 0.02 g of the absorber (1% on the weight of the mass of fibers), formula VI compound or in a bath containing 30 cm³ of water and 0.01 g (0.5% on the weight of the mass of the fibers) of formula VI or VII absorber. The application process was carried out at the temperature of 30°C. After 10 minutes, 1.5 g of crystalline sodium (VI) sulphate was added to application bath. Within 10 minutes the mixture was heated to 40° C, and then 0.6 g of sodium carbonate was added. Within 20 minutes, the bath temperature was raised to 60° C. Application process at this temperature was continued for additional 60 minutes, after which the fabric samples were rinsed, washed and dried. The values of the UPF coefficients of the samples after application bath solutions containing compounds of formula VI or VII are given in Table 1.

### Example 8 - Application of absorbers of the formula VI or VII - method 2.

A sample of cotton fabric with the mass of 2 g and surface density of 175 g/m², was placed in a bath containing 30 cm³ of water and 0.02 g of the absorber (1% on the weight of the mass of fibers) or in a bath containing 30 cm³ of water and 0.01 g (0.5% on the weight of the mass of the fibers) of formula VI or VII absorber. The application process was carried out at the temperature of 30° C. After 10 minutes, 1.5 g of crystalline sodium (VI) sulphate was added to application bath. Within 30 minutes the mixture was heated to 80° C, and then 0.6 g of sodium carbonate was added. Application process at this temperature was continued for additional 60 minutes, after which the fabric samples were rinsed, washed and dried. The values of the UPF coefficients of the samples after application bath in solutions containing compounds of formula VI or VII are given in Table 1.

**Table 1. The results of the measurements of UPF cotton knitted fabric after application of absorbers - formulas III - VII.**

| Absorber formula | The application method according to the example | UPF | |
|---|---|---|---|
| | | Concentration of the absorber 0.5% | Concentration of the absorber 1% |
| III | 6 | 99.6 | 108.9 |
| IV | 6 | 132.5 | 125.6 |
| V | 6 | 83.6 | 96.2 |
| VI | 7 | 81.5 | 129.6 |
| | 8 | 88.05 | 114.1 |
| VII | 7 | 81.7 | 90.3 |
| | 8 | 74.2 | 101.5 |

The resulting products of the invention arc highly soluble in water, characterized by a high molar absorption in the field of ultraviolet radiation from the UV-B range, good affinity for cellulose fibers and can he applied to these fibers in the same way as reactive dyes. When the substituent R in formula I represents a chlorine atom, the application process of the absorbers is the same as in the case of reactive dichlorotriazine dyes. When the substituent R in Formula I represents a methoxy group or a substituted or unsubstituted amino group, the application process of the absorbers is the same as in the case of reactive monochlorotriazine dyes. If the substituent R is the residue represented by formula III, such products can be applied in the same manner as dyeing with reactive vinylsulphone dyes or reactive monochlorotriazine dyes.

## Claims

1. Compounds of formula I: 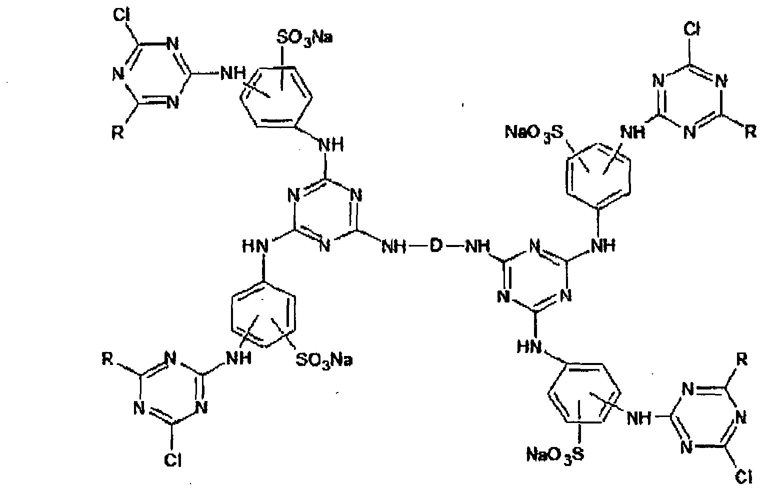 in which D represents a single bond or an aliphatic or aromatic diamine residue, R represents chlorine atom, methoxy residue, aminophenyl-sulphonic acid residue, aminophenyldisulphanic residue, ethanolamine or diethanolamine residue, morpholine residue, or residue represented by formula II:

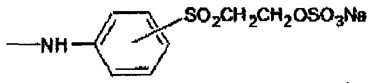

2. The method of production of compounds of formula I 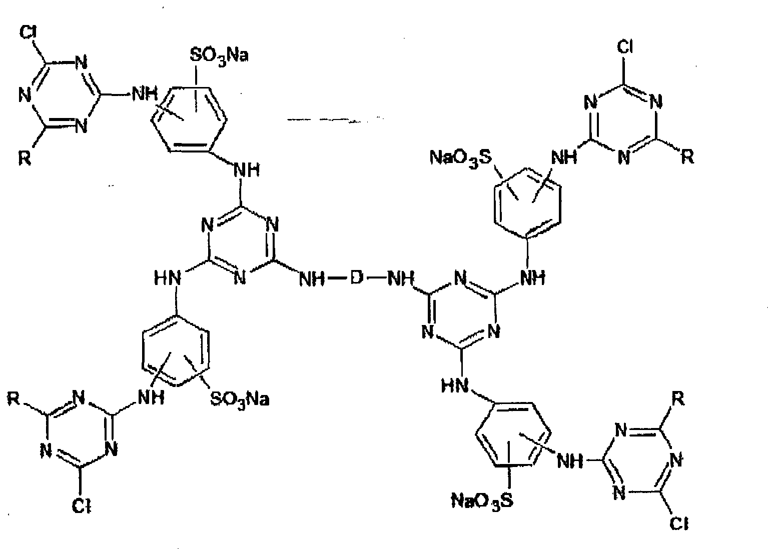 in which D represents a single bond or an aliphatic or aromatic diamine residue, R represents chlorine atom, methoxy residue, aminophenyl-sulphonic acid residue, aminophenyldisulfonic acid residue, ethanolamine or diethanolamine residue, morpholine residue, or residue represented by formula: 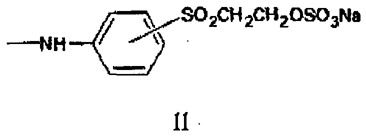 **characterized in that** dispersed 2,4,6-trichloro-1 ,3,5-triazine is condensed with aryl aminosulphonic acid in the ratio of one mole of acid per one mole of triazine, after which the product of the reaction is condensed with an additional mole of aryl diaminosulfonic acid and then with appropriate diamine, used in quantities of half mole of diamine per one mole of reaction product, and the final condensation product is reacted with at least four moles of chlorinated derivatives of symmetric 1,3,5-triazine.

3. Method according to claim 2 **characterized in that** aryl diaminosulphonic acid is replaced with diaminobenzene disulphonic acid.

4. Method according to claim 2 **characterized in that** diamine is replaced with amine selected from the group including p-phenylenediamine, o-phenylenediamine, hydrazine.

## Patentansprüche

1. Verbindungen mit der Formel I 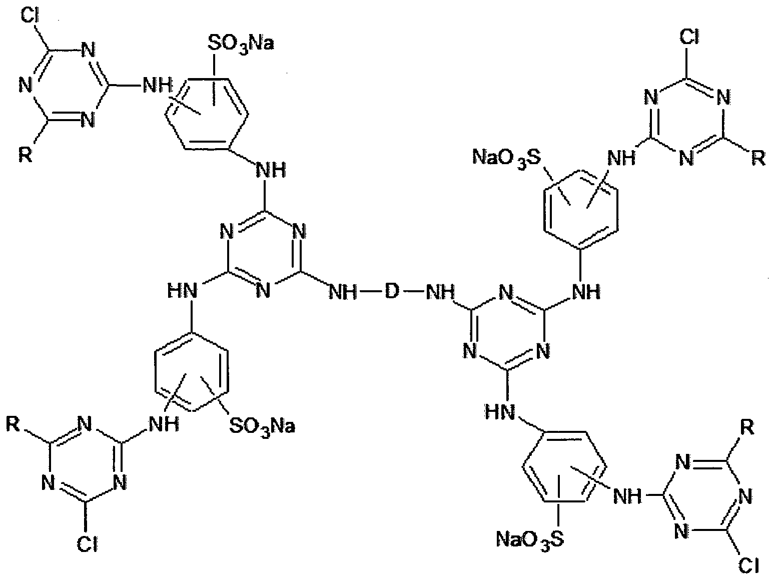 worin D eine einzelne Bindung oder einen aromatischen Ring darstellt, R ein Chloratom, einen Metoxyl-Rest, einen Aminophenylsulfonsäure-Rest, einen Aminophenyldisulfonsäure-Rest, einen Monoethanolamin-Rest, einen Diethanolamin-Rest, einen Morpholin-Rest oder einen Rest der Formel II 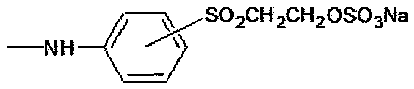 ist.

2. Verfahren zur Herstellung einer Verbindungen-der Formel 1 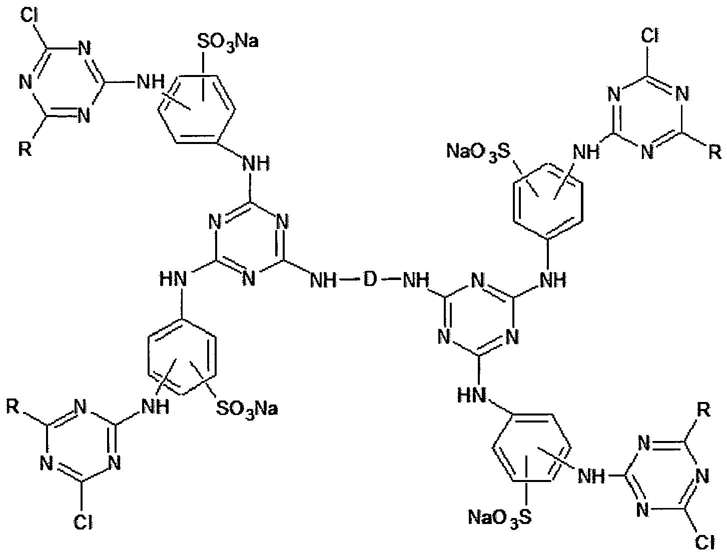 worin D eine einzelne Bindung oder einen aromatischen Ring darstellt, R ein Chloratom, einen Metoxyl-Rest, einen Aminophenylsulfonsäure-Rest, einen Aminophenyldisulfonsäure-Rest, einen Monoethanolamin-Rest, einen Diethanolamin-Rest, einen Morpholin-Rest oder einen Rest der Formel II 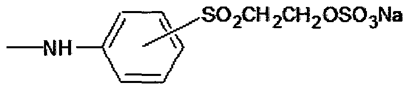 ist,
**dadurch gekennzeichnet, dass** dispergiere 2,4,6-trichloro-1,3,5-triazine der Kondensationsreaktion mit der Arylaminsulfonsäure im Verhältnis ein Mol Säure pro ein Mol Triazine unterzogen wird, und anschließend wird das Produkt dieser Reaktion mit einem weiteren Mol der Arylaminsulfonsäure kondensiert, und danach mit der aliphatischen oder aromatischen Diamin, die im Verhältnis ein halbes Mol Diamin pro ein Mol des Reaktionsproduktes verwendet wird, und dann wird das Produkt der letzten Kondensation einer Reaktion mit mindestens vier Molen der symmetrischen Chlorderivate der 1,3,5-triazin unterzogen.

3. Verfahren nach Anspruch 2, **dadurch gekennzeichnet, dass** als Arylaminsulfsonsäure die Diaminbenzensulfonsäure verwendet wird.

4. Verfahren nach Anspruch 2, **dadurch gekennzeichnet, dass** als Diamin eine Amin verwendet wird, die aus einer Gruppe ausgewählt wurde, die p-Phenylendiamin, o-Phenylendiamin, Hydrazin umfasst.

## Revendications

1. Les composés de la formule 1 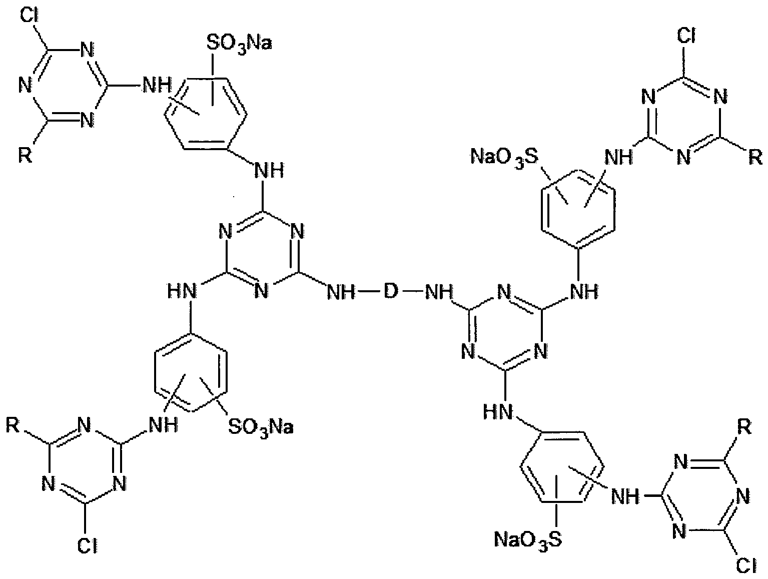 où D signifie une liaison simple ou un noyau aromatique, R signifie un atome de chlore, un résidu méthoxy, un résidu de l'acide amino-phényl-sulphonique, un résidu de l'acide amino-phényl-di-sulphonique, un résidu de l'éthanolamine ou de la diéthanolamine, un résidu de la morpholine ou un résidu représenté par la formule II :

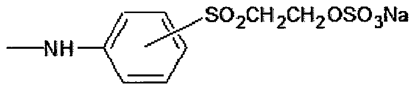

2. Le procédé de préparation des composés de la formule 1 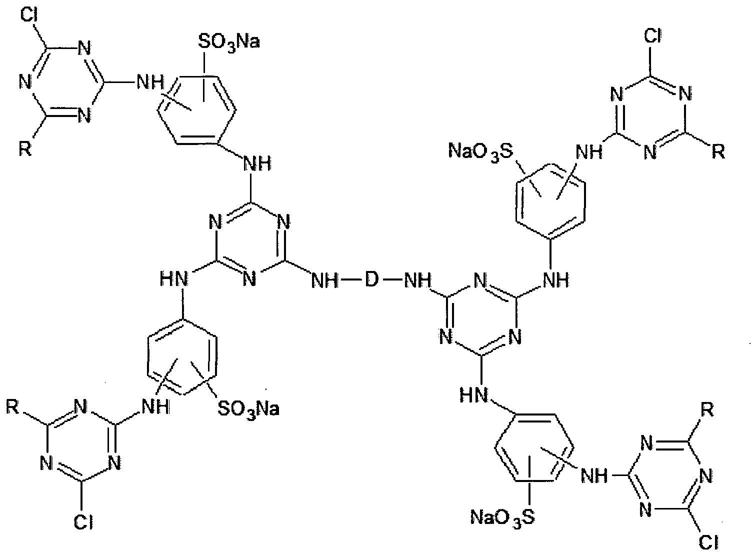 où D signifie une liaison simple ou un noyau aromatique, R signifie un atome de chlore, un résidu méthoxy, un résidu de l'acide amino-phényl-sulphonique, un résidu de l'acide amino-phényl-di-sulphonique, un résidu de l'éthanolamine ou de la diéthanolamine, un résidu de la morpholine ou un résidu représenté par la formule II : 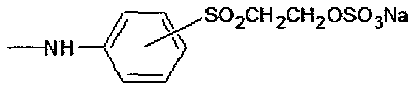 **Caractérisé en ce que** 2,4,6-trichloro-1,3,5-triazine dispersée est soumise à la réaction de consdensation avec un acide amino-aryl-sulfonique dans une proportion une mole de l'acide amino-aryl-sulfonique de triazine, mais le produit de la réaction est condensé avec une autre mole de l'acide amino aryl-sulfonique et ensuite avec la diamine aliphatique ou aromatique, utilisée en une quantité d'une demi-mole de diamine par une mole de produit de réaction, après quoi le produit de la dernière condensation est soumise à la réaction avec au moins quatre moles de dérivés chlorés de 1,3,5-triazine symétrique.

3. Le procédé selon la revendication 2 **caractérisé en ce que** l'acide diaminobenzènesulfonique est utilisé comme l'acide amino-aryl-sulfonique

4. Le procédé selon la revendication 2 **caractérisé en ce que** l'amine sélectionnée dans le groupe comprenant la p-phénylènediamine, la o-phénylènediamine, ou 1`hydrazine est utilisée comme la diamine.
